# EUROPEAN PATENT SPECIFICATION

(11) **EP 4 604 918 B1**
(45) Date of publication and mention of the grant of the patent: **02.12.2026**
(21) Application number: 23793850.1
(22) Date of filing: 20.10.2023
(51) Int. Cl.: A61K 9/00, A61K 9/08, A61K 9/10, A61K 47/14, A61K 47/10, A61K 31/426, A61P 35/00, A61P 35/02

(54) **COMPOSITIONS COMPRISING A BENZENE SULFONAMIDE THIAZOLE COMPOUND**
ZUSAMMENSETZUNGEN MIT EINER BENZENSULFONAMIDTHIAZOLVERBINDUNG
COMPOSITIONS COMPRENANT UN COMPOSÉ BENZÈNE SULFONAMIDE THIAZOLE

(30) Priority: 20.10.2022 EP 22306586
(43) Date of publication of application: 27.08.2025
(73) Proprietor: Biper Therapeutics SAS, 67000 Strasbourg (FR); Institut National de la Santé et de la Recherche Médicale (INSERM), 75013 Paris (FR); Université Côte d'Azur, 06103 Nice Cedex 2 (FR); Centre National de la Recherche Scientifique (CNRS), 75016 Paris (FR)
(72) Inventor: RONCO, Cyril, 06103 NICE CEDEX 2 (FR); BENHIDA, Rachid, 75016 Paris (FR); ROCCHI, Stephane, 75013 Paris (FR); CHELBI, Mehdi, 67000 Strasbourg (FR)
(74) Representative: Icosa
(86) International application number: PCT/EP2023/079371
(87) International publication number: WO 2024/084086

(56) References cited:
- WO-A1-2013/052845
- WO-A2-2009/137391
- US-A- 3 538 214
- US-B2- 9 567 310
- XU ET AL: "Endoplasmic Reticulum Stress Signaling as a Therapeutic Target in Malignant Pleural Mesothelioma", CANCERS, vol. 11, no. 10, 8 October 2019 (2019-10-08), pages 1502, XP093024014, DOI: 10.3390/cancers11101502
- STRICKLEY ET AL: "Solubilizing expients in oral and injectable formulations", ENVIRONMENTAL SCIENCE AND POLLUTION RESEARCH, SPRINGER BERLIN HEIDELBERG, BERLIN/HEIDELBERG, vol. 21, no. 2, 1 February 2004 (2004-02-01), pages 201 - 230, XP008136709, ISSN: 0724-8741, DOI: 10.1023/B:PHAM.0000016235.32639.23
- ALI SHAUKAT ET AL.: "Kolliphor® HS 15 - An Enabler for Parenteral and Oral Formulations", AMERICAN PHARMACEUTICAL REVIEW, 25 February 2019 (2019-02-25), pages 1 - 17, XP055827718, Retrieved from the Internet <URL:https://www.americanpharmaceuticalreview.com/Featured-Articles/358749-Kolliphor-HS-15-An-Enabler-for-Parenteral-and-Oral-Formulations/> [retrieved on 20210726]

## Description

### FIELD OF INVENTION

The present invention relates to pharmaceutical compositions comprising N-(4-(6-((5-(dimethylamino)naphthalene)-1-sulfonamido)pyridin-2-yl)thiazol-2-yl)acetamide, to their use as medicament and to their use in the prevention and/or treatment of cancers.

### BACKGROUND OF INVENTION

The compound of formula (I): 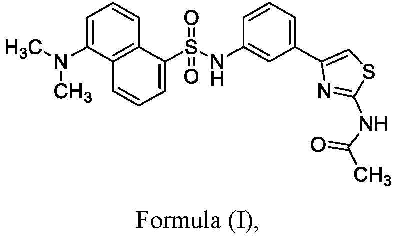 has been described for its anti-cancerous activity (Cerezo et al., Compounds Triggering ER Stress Exert Anti-Melanoma Effects and Overcome BRAF Inhibitor Resistance, Cancer Cell, 2016, 29, 1-15).

Example of prior art can be found in US 9,567,310, US 3,538,214, WO 2009/137391, WO 2013/052845, Xu et al., "Endoplasmic reticulum stress signaling as a therapeutic target in malignant pleural mesothelioma", Cancers, vol. 11, no. 10, 8 October 2019, page 1502, Strickley et al., "Solubilizing expients in oral and injectable formulations", ESPR, vol. 21, no. 2, 1 February 2004, pages 201-230 and Ali Shaukat et al., "Kolliphor® HS 15 - An enabler for parenteral and oral formulations", American pharmaceutical review, 25 February 2019, pages 1-17.

However, the bioavailability of said compound of formula (I) after administration of a composition according to the prior art comprising said compound by oral route is very low, of less than 3%. There is thus a need for enhancing the bioavailability of said compound of formula (I) for an administration by oral route.

The inventors of the present invention have surprisingly discovered that a specific composition comprising the compound of formula (I) allows the obtention of a better bioavailability after administration by oral route, in particular a bioavailability of more than 60%.

### SUMMARY

The present invention relates to a pharmaceutical composition comprising:
- a compound of formula (I) or a pharmaceutically acceptable salt, solvate or mixtures thereof, wherein formula (I) is as follows: 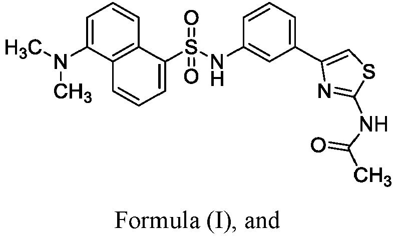
- from 5% v/v to 30% v/v, preferably from 10% v/v to 25% v/v, more preferably from 15% v/v to 25% v/v, even more preferably about 25% v/v, of at least one poly(ethylene glycol) selected from the group consisting of substituted poly(ethylene glycol), non-substituted poly(ethylene glycol) and mixtures thereof, the percentages being expressed relative to the total volume of the pharmaceutical composition,
- from 2% v/v to 10% v/v, preferably from 3% v/v to 7% v/v, more preferably from 4% v/v to 6% v/v, even more preferably about 5% v/v, of at least one solvent selected from the group consisting of ethanol, dimethyl sulfoxide, dimethylacetamide and mixtures thereof, the percentages being expressed relative to the total volume of the pharmaceutical composition, and
- water.

Advantageously, the at least one poly(ethylene glycol) is a mixture of at least one substituted poly(ethylene glycol) and at least one non-substituted poly(ethylene glycol). Preferably, the at least one poly(ethylene glycol) is a mixture of poly(ethylene glycol)(15)-hydroxystearate and poly(ethylene glycol) 400.

More advantageously, the non-substituted poly(ethylene glycol) represents from 5% v/v to 15% v/v, preferably from 7% v/v to 13% v/v, more preferably from 8% v/v to 12% v/v, even more preferably about 10% v/v, relative to the total volume of the pharmaceutical composition.

More advantageously, the substituted poly(ethylene glycol) represents from 10% v/v to 20% v/v, preferably from 12% v/v to 18% v/v, more preferably from 14% v/v to 16% v/v, even more preferably about 15% v/v, relative to the total volume of the pharmaceutical composition.

Advantageously, said pharmaceutical composition comprises from 60% v/v to 93% v/v, preferably from 68% v/v to 87% v/v, more preferably from 69% v/v to 81% v/v, even more preferably about 70% v/v, of water.

Advantageously, said pharmaceutical composition comprises from 60% v/v to 93% v/v, preferably from 68% v/v to 87% v/v, more preferably from 69% v/v to 71% v/v, even more preferably about 70% v/v, of water.

Advantageously, said pharmaceutical composition comprises from 1 mg/mL to 320 mg/mL of the compound of formula (I) or pharmaceutically acceptable salt, solvate or mixtures thereof.

Advantageously, the at least one solvent is ethanol.

Advantageously, said pharmaceutical composition further comprises at least one further excipient selected from the group consisting of: solvents, diluents carriers, fillers, bulking agents, binders, disintegrants, polymer, lubricant, glidant, surfactants, isotonic agents, thickening or emulsifying agents, stabilizers, absorption accelerators, flavoring agents, taste maskers, preservatives, antioxidants, buffering agents, gelling agent, solubilizing agent and combinations thereof.

The present invention also relates to the pharmaceutical composition according the invention, for use as a medicament.

The present invention also relates to the pharmaceutical composition according the invention, for use in the treatment or prevention of a cancer in a subject in need thereof.

Advantageously, said cancer is selected from the group consisting of: esophageal cancer, gastric cancer, intestinal cancer, colorectal cancer, pancreatic cancer and melanoma.

Advantageously, said cancer is a solid cancer. Preferably, the solid cancer is selected from the group consisting of: esophageal cancer, gastrointestinal cancer, gastric cancer, intestinal cancer, colorectal cancer, colon cancer, rectal cancer, pancreatic cancer, biliary tract cancer, liver cancer, kidney cancer, lung cancer, pleural cancer, urinary tract cancer, prostate cancer, endometrium cancer, ovarian cancer, breast cancer, autonomic ganglia cancer, salivary cancer, thyroid cancer, central neural system cancer, bone cancer, soft tissue cancer, lymphoma, skin cancer, carcinomas and melanoma. More preferably, the solid cancer is selected from the group consisting of: esophageal cancer, gastric cancer, intestinal cancer, colorectal cancer, colon cancer, rectal cancer, skin cancer and pancreatic cancer. More preferably, the solid cancer is selected from the group consisting of: esophageal cancer, gastric cancer, intestinal cancer, colorectal cancer, colon cancer, rectal cancer, and pancreatic cancer. Even more preferably, the solid cancer is selected from the group consisting of: colorectal cancer, colon cancer and rectal cancer. Better, the solid cancer is colorectal cancer.

Alternatively, said cancer is a liquid cancer. Preferably, the liquid cancer is selected from the group consisting of: lymphoma, leukemia and hematopoietic cancer. More preferably, the liquid cancer is leukemia. Even more preferably, the liquid cancer is chronic myelogenous leukemia.

Advantageously, said pharmaceutical composition is administered by a route of administration selected from the group consisting of: oral route, intraperitoneal route, intraspinal route, intraarterial route, intravenous route, intramuscular route and subcutaneous route. Preferably, said pharmaceutical composition is administered by oral route.

Advantageously, said pharmaceutical composition is administered once a day, twice a day, three times a day, four times a day or every 2 days, every 3 days, or 5 times a week.

More advantageously, said pharmaceutical composition is administered once a day, twice a day, three times a day or four times a day.

More advantageously, said pharmaceutical composition is administered every 2 days, every 3 days, or 5 times a week.

Advantageously, the dosage of the compound of formula (I) or a pharmaceutically acceptable salt, solvate or mixtures thereof, which is administered in one administration to the subject, ranges from 5 mg to 350 mg per kilogram of the subject weight, preferably from 15 mg to 35 mg per kilogram of the subject weight.

### DEFINITIONS

In the present invention, the following terms have the following meanings:
**"About",** before a figure or number, refers to plus or minus 10% of the face value of that figure or number. In one embodiment, "about", before a figure or number, refers to plus or minus 5% of the face value of that figure or number.
**"Active agent"** refers to an agent that has a therapeutic effect. The agent may be a chemical or a biological substance. Preferably, the active agent is a chemical substance. The therapeutic effect may be the prevention, delay, reduction in severity and/or frequency or suppression of at least one symptom associated with a pathological condition, or the prevention, slowing down or suppression of the underlying cause of a pathological condition, or the improvement or repair of a damage.
**"Bioavailability"** refers to the fraction of a drug that reaches systemic circulation unaltered after administration of said drug. Advantageously, the bioavailability F may be measured using the following formula: F(%)=[AUCinf(PO)*dose(IV)* 100]/[AUCinf(IV)*dose(PO)], wherein AUCinf(PO) and dose(PO) are the AUCinf obtained after the oral administration of the dose(PO) (PO=per os) and wherein AUCinf(IV) and dose(IV) are the AUCinf obtained after the intravenous administration of the dose(IV) (IV= intravenous).
**"Buffer"** relates to a mixture of a weak acid and its conjugate base, or a weak base and its conjugate acid, allowing the pH of a pharmaceutical composition to be maintained constant when a small amount of strong acid or base is added thereto. **"Buffering agent"** refers to the specific chemical that is present in both an acidic and a basic forms in a buffer, allowing the pH of a pharmaceutical composition to be maintained constant.
**"Cancer"** refers to any disease caused by the transformation of cells that become abnormal and proliferate excessively. **"Solid cancer"** refers to a cancer in which the abnormal and proliferating cells are located in solid organ such as breast or prostate. Advantageously, a solid cancer may be selected from the group consisting of: esophageal cancer, gastrointestinal cancer, stomach cancer, intestinal cancer, colorectal cancer, colon cancer, rectal cancer, pancreatic cancer, biliary tract cancer, liver cancer, kidney cancer, lung cancer, pleural cancer, urinary tract cancer, prostate cancer, endometrium cancer, ovarian cancer, breast cancer, autonomic ganglia cancer, salivary cancer, thyroid cancer, central neural system cancer, bone cancer, soft tissue cancer, lymphomas, skin cancer, carcinoma and melanoma. More advantageously, a solid cancer may be selected from the group consisting of: stomach cancer, esophageal cancer, and skin cancer. The skin cancer may be selected from the group consisting of melanoma and carcinoma. More advantageously, a solid cancer may be selected from the group consisting of: colorectal cancer, colon cancer and rectal cancer. **"Liquid cancer"** refers to a cancer in which the abnormal and proliferating cells are located in a liquid body fluid, for example in the blood (i.e. leukemia) or in the lymph. Advantageously, a liquid cancer may be selected from the group consisting of: lymphoma, leukemia and hematopoietic cancer. Preferably, a liquid cancer may be leukemia. More preferably, a liquid cancer may be chronic myelogenous leukemia.
**"Colloidal"** refers to a state of subdivision, implying that the molecules or polymolecular particles dispersed in a medium have at least in one direction a dimension roughly between 1 nm and 1 µm, or that in a system discontinuities are found at distances of that order.
**"Comprising" or "comprise"** is to be construed in an open, inclusive sense, but not limited to.
**"Consisting of" or "consist"** is to be construed in a close, non-inclusive sense, limited to the features following this term.
**"Dose"** refers to the amount of active agent administered at one time. In one embodiment, two oral doses are administered to one subject from 6 hours to 1 week apart, preferably from 8 hours to 48 hours apart, more preferably from 12 hours to 24 hours apart, even more preferably about 24 hours apart. Advantageously, a dose is a human dose. A human dose is a standard human dose for a man weighing 70 kg.
**"Emulsion"** refers to a fluid colloidal system in which liquid droplets are dispersed in a liquid. The droplets often exceed the usual limits for colloids in size. A **"simple emulsion"** is denoted by the symbol O/W (or by the term oil-in-water) if the continuous phase is an aqueous solution (=aqueous phase) and by W/O (or by the term water-in-oil) if the continuous phase is an organic liquid (an "oil"). **"Double emulsions"** are more complicated emulsion, such as W/O/W (also named water-in-oil-in-water double emulsion, i.e. aqueous droplets contained within oil droplets dispersed in a continuous aqueous phase). In a W(1)/O/W(2) double emulsion, the internal emulsion refers to the emulsion of the innermost aqueous phase W(1) in the oil phase O; and the external emulsion refers to the emulsion of the oil phase O in the external aqueous phase W(2).
**"Excipient"** refers to any inactive ingredient, which is required for the formulation of an active agent in a suitable dosage form. In one embodiment, "excipient" refers to any and all solvents, diluents carriers, fillers, bulking agents, binders, disintegrants, polymer, lubricant, glidant, surfactants, isotonic agents, thickening or emulsifying agents, stabilizers, absorption accelerators, flavoring agents, taste maskers, preservatives, antioxidants, buffering agents, gelling agent, solubilizing agent or any combination thereof.
**"From X to Y"** refers to the range of values between X and Y, the limits X and Y being included in said range.
**"Oil phase"** refers to a phase not miscible with an aqueous phase, which means that the amount by weight of an aqueous phase capable of being solubilized in the oil phase is less than or equal to 5%, preferably less than 1%, more preferably less than 0, 5%, even preferably of 0%, based on the total weight of the oil phase, and that the amount (by weight) of the oil phase capable of being solubilized in an aqueous phase is less than or equal to 5%, preferably less than 1%, more preferably less than 0.5%, even preferably of 0%, based on the total weight of the aqueous phase. The oil phase does not necessarily contain oil. The oil phase has typically a viscosity greater than the water viscosity.
**"Pharmaceutical composition"** refers to the combination of at least one active agent and at least one pharmaceutically acceptable excipient.
**"Pharmaceutically acceptable"** refers to generally safe, non-toxic and neither biologically nor physiologically nor otherwise undesirable for mammalian animals, in particular for humans, dogs, cats and non-human primate animals.
**"Pharmacokinetic parameters":** among the pharmacokinetic parameters, **"Cmax"** refers to the maximum observed plasmatic concentration, **"Tmax"** refers to the time required to reach the maximum observed Cmax plasmatic concentration, **"T1/2"** refers to the half-life time, i.e., the time at which the Cmax plasmatic concentration has been halved (during the elimination phase), **"Cl/F"** refers to the renal clearance, **"Vz/F"** refers to the volume of distribution (elimination phase), and **"AUC inf PO"** refers to the area under the curve (corresponding to the exposure of a subject to the compound tested).
**"Poly(ethylene glycol)(15)-hydroxystearate"** refers to the molecule whose CAS number is 70142-34-6 and which has the following formula: 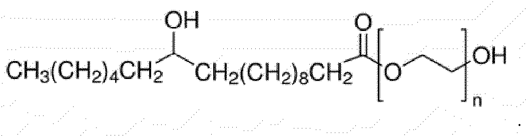 For example, the poly(ethylene glycol)(15)-hydroxystearate may be the product marketed under the name Kolliphor^{®} HS 15.
**"Salt of a compound"** refers to acid or base addition salts of said compound. The acid addition salts are formed with pharmaceutically acceptable organic or inorganic acids; the base addition salts are formed when an acid proton present in the compound is either replaced by a metal ion or coordinated with a pharmaceutically acceptable organic or inorganic base. In one embodiment, the acid addition salt is selected from the group consisting of acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts. In one embodiment, the base addition salt is selected from the group consisting of aluminium, arginine, benzathine, calcium, choline, diethylamine, 2-(diethylamino)ethanol, diolamine, ethanolamine, glycine, 4-(2-hydroxyethyl)-morpholine, lysine, magnesium, meglumine, morpholine, olamine, potassium, sodium, tromethamine and zinc salts.
**"Solvate of a compound"** refers to a molecular complex comprising said compound and one or more pharmaceutically acceptable solvent molecules. **"Hydrate of a compound"** refers to a molecular complex comprising the compound and one or more pharmaceutically acceptable solvent molecules, wherein the solvent is water.
**"Subject" or "patient"** refers to a mammalian animal, wherein **"mammalian animal"** refers to a human or a non-human mammalian animal. In one embodiment, "subject" refers to a human (man or woman). In one embodiment, "subject" refers to a non-human mammalian animal, preferably to a non-human mammalian animal selected from the group consisting of a cat, a dog, a horse and a non-human primate such as a monkey.
**"Surfactant"** refers to a substance which lowers the surface tension of the medium in which it is dissolved, and/or the interfacial tension with other phases, and, accordingly, is positively adsorbed at the liquid/vapor, liquid/liquid and/or at other interfaces.
**"Suspension"** refers to a liquid in which solid particles are dispersed.
**"Therapeutically effective amount" or "effective amount"** of an active agent or of a composition refers to a nontoxic but sufficient amount of said active agent or composition to provide the desired therapeutic effect.
**"Treating" or "treatment"** refers to any action which makes it possible to prevent, delay, reduce in severity and/or frequency or suppress at least one symptom associated with a pathological condition, or to prevent, slow down or suppress the underlying cause of a pathological condition, or the improvement or remediation of damage. In one embodiment, **"treatment"** refers to a curative treatment. In another embodiment, **"treatment"** refers to a preventive treatment. In another embodiment, **"treatment"** refers to a preventive and/or curative treatment.
**"Ultrapure water"** refers to water that has been purified to uncommonly stringent specifications and treated to the highest levels of purity for all contaminant types, including: organic and inorganic compounds, dissolved and particulate compounds, volatile and non-volatile compounds, reactive and inert compounds, hydrophilic and hydrophobic compounds, and dissolved gaseous compounds.

### DETAILED DESCRIPTION

### Pharmaceutical composition

The present invention relates to a pharmaceutical composition comprising:
- a compound of formula (I) or a pharmaceutically acceptable salt, solvate or mixtures thereof, wherein formula (I) is as follows: 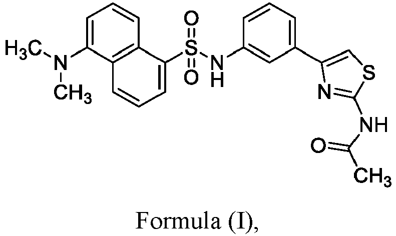
- from 5% v/v to 30% v/v, preferably from 10% v/v to 25% v/v, more preferably from 15% v/v to 25% v/v, even more preferably about 25% v/v, of at least one poly(ethylene glycol) selected from the group consisting of substituted poly(ethylene glycol)s, non-substituted poly(ethylene glycol)s and mixtures thereof, the percentages being expressed relative to the total volume of the pharmaceutical composition,
- from 2% v/v to 10% v/v, preferably from 3% v/v to 7% v/v, more preferably from 4% v/v to 6% v/v, even more preferably about 5% v/v, of at least one solvent selected from the group consisting of: ethanol, dimethyl sulfoxide, dimethylacetamide and mixtures thereof, the percentages being expressed relative to the total volume of the pharmaceutical composition, and
- water.

Advantageously, the at least one poly(ethylene glycol) is selected from the group consisting of substituted poly(ethylene glycol)s and mixtures thereof. More advantageously, the at least one poly(ethylene glycol) is selected from the group consisting of fatty acid derived poly(ethylene glycol)s and mixtures thereof. Preferably, the at least one poly(ethylene glycol) is poly(ethylene glycol)(15)-hydroxystearate. Preferably, the at least one substituted poly(ethylene glycol), in particular the poly(ethylene glycol)(15)-hydroxystearate, represents from 10% v/v to 20% v/v, more preferably from 12% v/v to 18% v/v, even more preferably from 14% v/v to 16% v/v, better about 15% v/v, relative to the total volume of the pharmaceutical composition.

Advantageously, the at least one poly(ethylene glycol) is selected from the group consisting of non-substituted poly(ethylene glycol)s and mixtures thereof. More advantageously, the at least one poly(ethylene glycol) is selected from the group consisting of poly(ethylene glycol) 200, poly(ethylene glycol) 400, poly(ethylene glycol) 600, poly(ethylene glycol) 800, poly(ethylene glycol) 1000, poly(ethylene glycol) 4000 and mixtures thereof. Even more advantageously, the at least one poly(ethylene glycol) is selected from the group consisting of poly(ethylene glycol) 200, poly(ethylene glycol) 400, poly(ethylene glycol) 600 and mixtures thereof. Preferably, the at least one poly(ethylene glycol) is poly(ethylene glycol) 400. Preferably, the at least one non-substituted poly(ethylene glycol), in particular the poly(ethylene glycol) 400, represents from 5% v/v to 15% v/v, more preferably from 7% v/v to 13% v/v, even more preferably from 8% v/v to 12% v/v, better about 10% v/v, relative to the total volume of the pharmaceutical composition.

Advantageously, the at least one poly(ethylene glycol) is selected from the group consisting of fatty acid derived poly(ethylene glycol)s, non-substituted poly(ethylene glycol)s and mixtures thereof. More advantageously, the at least one poly(ethylene glycol) is selected from the group consisting of fatty acid derived poly(ethylene glycol)s, poly(ethylene glycol) 200, poly(ethylene glycol) 400, poly(ethylene glycol) 600, poly(ethylene glycol) 800, poly(ethylene glycol) 1000, poly(ethylene glycol) 4000, poly(ethylene glycol) 6000 and mixtures thereof. More advantageously, the at least one poly(ethylene glycol) is selected from the group consisting of fatty acid derived poly(ethylene glycol)s, poly(ethylene glycol) 200, poly(ethylene glycol) 400, poly(ethylene glycol) 600, poly(ethylene glycol) 800, poly(ethylene glycol) 1000, poly(ethylene glycol) 4000 and mixtures thereof. More advantageously, the at least one poly(ethylene glycol) is selected from the group consisting of fatty acid derived poly(ethylene glycol)s, poly(ethylene glycol) 200, poly(ethylene glycol) 400, poly(ethylene glycol) 600 and mixtures thereof.

More advantageously, the at least one poly(ethylene glycol) is selected from the group consisting of poly(ethylene glycol)(15)-hydroxystearate, fatty acid derived poly(ethylene glycol)s other than poly(ethylene glycol)(15)-hydroxystearate, non-substituted poly(ethylene glycol)s and mixtures thereof. More advantageously, the at least one poly(ethylene glycol) is selected from the group consisting of poly(ethylene glycol)(15)-hydroxystearate, fatty acid derived poly(ethylene glycol)s other than poly(ethylene glycol)(15)-hydroxystearate, poly(ethylene glycol) 200, poly(ethylene glycol) 400, poly(ethylene glycol) 600, poly(ethylene glycol) 800, poly(ethylene glycol) 1000, poly(ethylene glycol) 4000 and mixtures thereof. More advantageously, the at least one poly(ethylene glycol) is selected from the group consisting of poly(ethylene glycol)(15)-hydroxystearate, fatty acid derived poly(ethylene glycol)s other than poly(ethylene glycol)(15)-hydroxystearate, poly(ethylene glycol) 200, poly(ethylene glycol) 400, poly(ethylene glycol) 600 and mixtures thereof.

More advantageously, the at least one poly(ethylene glycol) is selected from the group consisting of poly(ethylene glycol)(15)-hydroxystearate, poly(ethylene glycol) 200, poly(ethylene glycol) 400, poly(ethylene glycol) 600, poly(ethylene glycol) 800, poly(ethylene glycol) 1000, poly(ethylene glycol) 4000 and mixtures thereof. More advantageously, the at least one poly(ethylene glycol) is selected from the group consisting of poly(ethylene glycol)(15)-hydroxystearate, poly(ethylene glycol) 200, poly(ethylene glycol) 400, poly(ethylene glycol) 600 and mixtures thereof.

Advantageously, the at least one poly(ethylene glycol) is a mixture of at least one substituted poly(ethylene glycol) and at least one non-substituted poly(ethylene glycol). More advantageously, the at least one poly(ethylene glycol) is a mixture of poly(ethylene glycol)(15)-hydroxystearate and poly(ethylene glycol) 400. Preferably:
- the substituted poly(ethylene glycol), in particular the poly(ethylene glycol)(15)-hydroxystearate, represents from 10% v/v to 20% v/v, more preferably from 12% v/v to 18% v/v, even more preferably from 14% v/v to 16% v/v, better about 15% v/v, relative to the total volume of the pharmaceutical composition; and
- the non-substituted poly(ethylene glycol), in particular the poly(ethylene glycol) 400, represents from 5% v/v to 15% v/v, more preferably from 7% v/v to 13% v/v, even more preferably from 8% v/v to 12% v/v, better about 10% v/v, relative to the total volume of the pharmaceutical composition.

Advantageously, the at least one solvent is dimethyl sulfoxide. Preferably, the pharmaceutical composition comprises from 2% v/v to 10% v/v, more preferably from 3% v/v to 7% v/v, even more preferably from 4% v/v to 6% v/v, better about 5% v/v, of dimethyl sulfoxide.

Advantageously, the at least one solvent is dimethylacetamide. Preferably, the pharmaceutical composition comprises from 2% v/v to 10% v/v, more preferably from 3% v/v to 7% v/v, even more preferably from 4% v/v to 6% v/v, better about 5% v/v, of dimethylacetamide.

More advantageously, the at least one solvent is ethanol. Preferably, the pharmaceutical composition comprises from 2% v/v to 10% v/v, more preferably from 3% v/v to 7% v/v, even more preferably from 4% v/v to 6% v/v, better about 5% v/v, of ethanol.

The ethanol may be used whatever its alcohol by volume (=ABV). Advantageously, the ethanol may be an ethanol solution of at least 50% ABV, preferably an ethanol solution of at least 70% ABV, more preferably an ethanol solution of at least 90% ABV, even more preferably an ethanol solution of at least 96% ABV (also named technical grade ethanol), better an ethanol solution of at least 99% ABV (also named absolute grade ethanol). In one embodiment, the ethanol may be an ethanol solution of 96% ABV.

### Advantageously, the water is ultrapure water.

Advantageously, said pharmaceutical composition comprises water in an amount *quantum satis* 100% v/v of the pharmaceutical composition. Preferably, said pharmaceutical composition comprises from 60% v/v to 93% v/v, preferably from 68% v/v to 87% v/v, more preferably from 69% v/v to 81% v/v, even more preferably about 70% v/v, of water.

Advantageously, said pharmaceutical composition comprises from 1 mg/mL to 500 mg/mL, preferably from 1 mg/mL to 350 mg/mL, more preferably from 1 mg/mL to 320 mg/mL, even more preferably from 50 mg/mL to 320 mg/mL, better about 150 mg/mL, of the compound of formula (I) or pharmaceutically acceptable salt, solvate or mixtures thereof. For clarity's sake, "1 mg/mL of the compound of formula (I)" refers to 1 milligram of the compound of formula (I) per 1 milliliter of the pharmaceutical composition.

Advantageously, said pharmaceutical composition comprises about 150 mg/mL of the compound of formula (I) or pharmaceutically acceptable salt, solvate or mixtures thereof.

In one embodiment, the pharmaceutical composition comprises:
- the compound of formula (I) or a pharmaceutically acceptable salt, solvate or mixtures thereof, wherein formula (I) is as described above,
- from 10% v/v to 20% v/v, more preferably from 12% v/v to 18% v/v, even more preferably from 14% v/v to 16% v/v, better about 15% v/v, of poly(ethylene glycol)(15)-hydroxystearate, the percentages being expressed relative to the total volume of the pharmaceutical composition,
- from 5% v/v to 15% v/v, more preferably from 7% v/v to 13% v/v, even more preferably from 8% v/v to 12% v/v, better about 10% v/v, of poly(ethylene glycol) 400, the percentages being expressed relative to the total volume of the pharmaceutical composition,
- from 2% v/v to 10% v/v, preferably from 3% v/v to 7% v/v, more preferably from 4% v/v to 6% v/v, even more preferably about 5% v/v, of ethanol, the percentages being expressed relative to the total volume of the pharmaceutical composition, and
- water.

In a preferred embodiment, the pharmaceutical composition comprises:
- from 1 mg/mL to 320 mg/mL of a compound of formula (I) or a pharmaceutically acceptable salt, solvate or mixtures thereof, wherein formula (I) is as described above,
- about 15% v/v of poly(ethylene glycol)(15)-hydroxystearate relative to the total volume of the pharmaceutical composition,
- about 10% v/v of poly(ethylene glycol) 400 relative to the total volume of the pharmaceutical composition,
- about 5% v/v of ethanol relative to the total volume of the pharmaceutical composition, and
- *quantum satis* 100% v/v of water.

In a more preferred embodiment, the pharmaceutical composition consists of:
- from 1 mg/mL to 320 mg/mL of a compound of formula (I) or a pharmaceutically acceptable salt, solvate or mixtures thereof, wherein formula (I) is as described above,
- about 15% v/v of poly(ethylene glycol)(15)-hydroxystearate relative to the total volume of the pharmaceutical composition,
- about 10% v/v of poly(ethylene glycol) 400 relative to the total volume of the pharmaceutical composition,
- about 5% v/v of ethanol relative to the total volume of the pharmaceutical composition, and
- about 70% v/v of water relative to the total volume of the pharmaceutical composition.

Advantageously, the pharmaceutical composition is an emulsion. The emulsion may be selected from the group consisting of a simple emulsion and a double emulsion. More advantageously, the pharmaceutical composition is a simple emulsion.

### Advantageously, the pharmaceutical composition is a suspension.

Advantageously, the pharmaceutical composition is a solution, preferably a clear solution.

Advantageously, said pharmaceutical composition is for oral administration. In other words, advantageously, said pharmaceutical composition is an oral pharmaceutical composition.

Advantageously, said pharmaceutical composition further comprises at least one further excipient selected from the group consisting of: solvents, diluents carriers, fillers, bulking agents, binders, disintegrants, polymer, lubricant, glidant, surfactants, isotonic agents, thickening or emulsifying agents, stabilizers, absorption accelerators, flavoring agents, taste maskers, preservatives, antioxidants, buffering agents, gelling agent, solubilizing agent and combinations thereof. More advantageously, said pharmaceutical composition further comprises at least one flavoring agents and/or taste maskers. Preferably, when the further excipients are present in the pharmaceutical composition according to the invention, said further excipients are present in general in an amount each of from 0.001% to 5% by weight, preferably from 0.001% to 1% by weight, relative to the total weight of the pharmaceutical composition. Needless to say, a person skilled in the art will take care to select these optional excipients such that the advantageous properties intrinsically associated with the pharmaceutical composition of the invention are not, or are not substantially, adversely affected by the envisaged excipient(s).

### Pharmaceutical composition for use as a medicament

The present invention also relates to the pharmaceutical composition according to the invention as described above for use as a medicament in a subject in need thereof.

The present invention also relates to a method of treating and/or preventing a disease by administering to a subject in need thereof an effective amount of the pharmaceutical composition according to the invention as described above.

The present invention also relates to the use of the pharmaceutical composition according to the invention as described above for the manufacture of a medicament.

The present invention also relates to the use of the pharmaceutical composition according to the invention as described above for the treatment and/or prevention of a disease in a subject in need thereof.

In the present application, a medicament may be a human medicament or a veterinary medicament.

### Pharmaceutical composition for use in the treatment or prevention of diseases

The present invention also relates to the pharmaceutical composition according to the invention as described above for use in the treatment and/or prevention of a cancer in a subject in need thereof.

The present disclosure also relates to a method of treating and/or preventing a cancer by administering to a subject in need thereof an effective amount of the pharmaceutical composition according to the invention as described above. Any references to methods of treatment by therapy or surgery or *in vivo* diagnosis methods of this description is to be interpreted as a reference to compounds, pharmaceutical compositions and medicaments of the present invention for use in those methods.

The present invention also relates to the use of the pharmaceutical composition according to the invention as described above for the manufacture of a medicament for the treatment and/or prevention of a cancer in a subject in need thereof.

The present invention also relates to the use of the pharmaceutical composition according to the invention as described above for the treatment and/or prevention of a cancer in a subject in need thereof.

Advantageously, the cancer is a solid cancer.

Advantageously, the cancer is selected from the group consisting of: esophageal cancer, gastric cancer, intestinal cancer, colorectal cancer, pancreatic cancer and melanoma. More advantageously, the cancer is selected from the group consisting of: colorectal cancer and melanoma. Even more advantageously, the cancer is colorectal cancer.

Advantageously, the cancer may be selected from the group consisting of: esophageal cancer, gastrointestinal cancer, stomach cancer, intestinal cancer, colorectal cancer, colon cancer, rectal cancer, pancreatic cancer, biliary tract cancer, liver cancer, kidney cancer, lung cancer, pleural cancer, urinary tract cancer, prostate cancer, endometrium cancer, ovarian cancer, breast cancer, autonomic ganglia cancer, salivary cancer, thyroid cancer, central neural system cancer, bone cancer, soft tissue cancer, lymphoma, skin cancer, carcinoma and melanoma. More advantageously, the cancer may be selected from the group consisting of: stomach cancer, esophageal cancer, and skin cancer. The skin cancer may be selected from the group consisting of melanoma and carcinoma. More advantageously, the cancer may be selected from the group consisting of: colorectal cancer, colon cancer and rectal cancer.

More advantageously, the cancer may be colorectal cancer.

More advantageously, the cancer may be colon cancer.

More advantageously, the cancer may be rectal cancer.

More advantageously, the cancer may be melanoma.

Advantageously, the pharmaceutical composition is administered by a route of administration selected from the group consisting of: oral route, intraperitoneal route, intraspinal route, intraarterial route, intravenous route, intramuscular route and subcutaneous route. More advantageously, said pharmaceutical composition is administered by oral route.

Advantageously, the pharmaceutical composition is administered once a day, twice a day, three times a day or four times a day. More advantageously, the pharmaceutical composition is administered by oral route once a day, twice a day, three times a day or four times a day.

Advantageously, the pharmaceutical composition is administered every day, every 2 days, every 3 days, or 5 times a week. More advantageously, the pharmaceutical composition is administered by oral route every day, every 2 days, every 3 days, or 5 times a week.

Advantageously, the pharmaceutical composition is administered, in particular by oral route, every day, every 2 days, every 3 days, or 5 times a week, wherein during a day of administration, said pharmaceutical composition is administered once a day, twice a day, three times a day or four times a day.

Advantageously, the dosage of the compound of formula (I) or a pharmaceutically acceptable salt, solvate or mixtures thereof, which is administered in one administration to the subject, ranges from 5 mg to 350 mg, preferably from 5 mg to 250 mg, more preferably about 150 mg, per kilogram of the subject's weight. More advantageously, the dosage of the compound of formula (I) or a pharmaceutically acceptable salt, solvate or mixtures thereof, which is administered in one administration to the subject by oral route, ranges from 5 mg to 350 mg, preferably from 5 mg to 250 mg, more preferably about 150 mg per kilogram of the subject's weight.

Advantageously, the dosage of the compound of formula (I) or a pharmaceutically acceptable salt, solvate or mixtures thereof, which is administered in one administration to the subject, ranges from 5 mg to 50 mg, preferably from 15 mg to 35 mg, per kilogram of the subject's weight. More advantageously, the dosage of the compound of formula (I) or a pharmaceutically acceptable salt, solvate or mixtures thereof, which is administered in one administration to the subject by oral route, ranges from 5 mg to 50 mg, preferably from 15 mg to 35 mg, per kilogram of the subject's weight.

Advantageously, the subject is a human, preferably a human over the age of 18, preferably over the age of 40, more preferably over the age of 50, even more preferably over the age of 65.

### BRIEF DESCRIPTION OF THE DRAWINGS

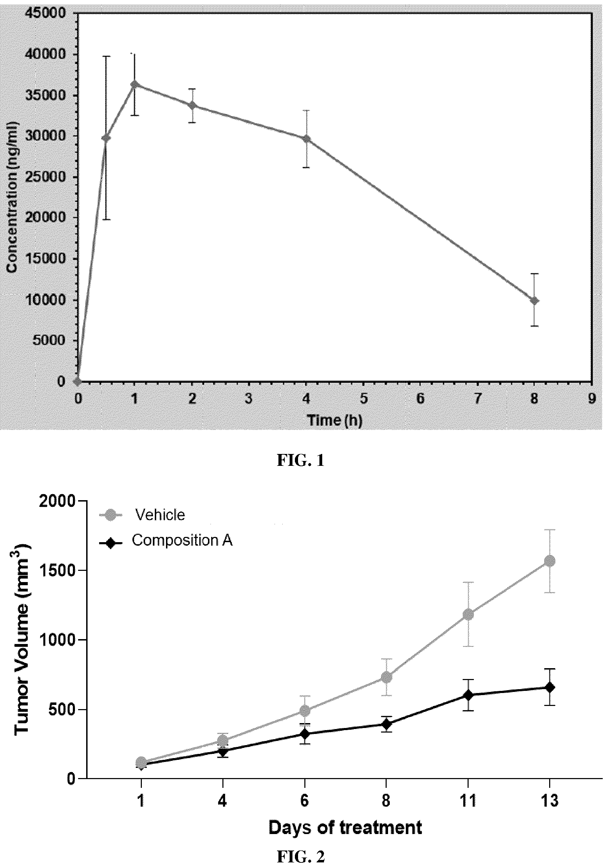

**Figure 1** is a graph showing the mean plasma concentration of the compound of formula (I) according to the invention as a function of time, following an oral administration of a pharmaceutical composition according to the invention to 3 swiss albino mice at a dosage of 150 mg of the compound of formula (I) per kilogram of each mouse.
**Figure 2** is a graph showing the tumor volume as a function of time, following an oral administration of either a pharmaceutical composition according to the invention to 10 BALB/C mice having a CT26 (Colorectal) tumor at a dosage of 150 mg of the compound of formula (I) per kilogram of each mouse, or a placebo to 10 BALB/C mice having a CT26 tumor.

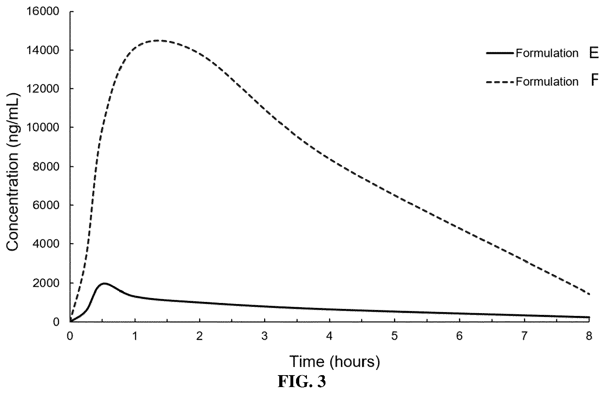

**Figure 3** is a graph showing the mean plasma concentration of the compound of formula (I) according to the invention as a function of time, following an oral administration of either a pharmaceutical composition F according to the invention to 3 swiss albino mice at a dosage of 150 mg of the compound of formula (I) per kilogram of each mouse, or a pharmaceutical composition E according to the prior art to 3 swiss albino mice at a dosage of 150 mg of the compound of formula (I) per kilogram of each mouse.

### EXAMPLES

The present invention is further illustrated by the following examples. In all examples, the bioavailability F was measured using the following formula $F \left(%\right) = \left[AUCinf\left(PO\right)*dose\left(IV\right)*100\right] / \left[AUCinf\left(IV\right)*dose\left(PO\right)\right] .$

### Example 1: Preparation of two compositions according to the invention

### Composition A according to the invention

An organic vehicle was prepared, comprising: 50% v/v of Kolliphor^{®} HS15, 33.33% v/v of PEG400 and 16.67% v/v of ethanol. Thus, in 1.940 mL of said organic vehicle, there was 0.9700 mL of Kolliphor^{®} HS15, 0.6466 mL of PEG400 and 0.3234 mL of ethanol.

96.99 mg of the compound of formula (I) according to the invention was weighed on a microbalance into a Wheaton vial. The compound was dissolved in 1.9400 mL of the organic vehicle described above (corresponding to 3 parts of the final vehicle volume) and the dosing solution was mixed well using a magnetic bar for approximately 30 minutes. Following this, the final vehicle component, 4.5266 mL of ultrapure water (corresponding to 7 parts of the final vehicle volume), was added slowly to the Wheaton vial and mixed well by inversion. The final dosing composition was a slight turbid fluorescent green/yellow solution and comprised 15 mg of the compound of formula (I) per milliliter of the solution.

### Composition B according to the invention

An organic vehicle was prepared, comprising: 50% v/v of Kolliphor^{®} HS15, 33.33% v/v of PEG400 and 16.67% v/v of ethanol. Thus, in 1.940 mL of said organic vehicle, there was 0.9700 mL of Kolliphor^{®} HS15, 0.6466 mL of PEG400 and 0.3234 mL of ethanol.

206.93 mg of the compound of formula (I) according to the invention was weighed on a microbalance into a Wheaton vial. The compound was dissolved in 1.9400 mL of the organic vehicle described above (corresponding to 3 parts of the final vehicle volume) and the dosing solution was mixed well using a magnetic bar for approximately 30 minutes. Following this, the final vehicle component, 4.5266 mL of ultrapure water (corresponding to 7 parts of the final vehicle volume), was added slowly to the Wheaton vial and mixed well by inversion. The final dosing composition was a green to yellow fine suspension and comprised 32 mg of the compound of formula (I) per milliliter of the suspension.

### Example 2: Evaluation of the bioavailability of the compound of formula (I) after administration of the composition A according to the invention prepared in Example 1

### Materials and Methods

The composition A according to the invention prepared in Example 1 was administered as a single dose by oral route to 3 male swiss albino mice at a dosage of 150 mg/kg for each mouse (corresponding to a volume dosage of 10 mL/kg by oral route). The mice were dosed orally through gastric gavage needle.

In addition, a composition named composition IV was administered as a single dose by intravenous route to 3 other male swiss albino mice at a dosage of 1 mg/kg for each mouse (corresponding to a volume dosage of 10 mL/kg by intravenous route). Composition IV contained 0.1 mg of the compound of formula (I) according to the invention per milliliter of said composition IV, and a vehicle containing 10% v/v of Kolliphor^{®} EL and 90% v/v of a saline solution (=an aqueous solution comprising 0.9% w/v of NaCl). Composition IV was prepared as follows: Kolliphor^{®} EL and saline solution were pre-mixed in the appropriate proportion (1/9, v/v), and then the compound of formula (I) according to the invention was dissolved in the requested volume to obtain the composition IV.

For each of the six mice, blood samples were collected in the saphenous vein at different endpoints (T0+0.5hour, T0+1hour, T0+2hours, T0+4hours and T0+8hours following the oral or intravenous administration at T0) and centrifugated to separate plasma. The plasmatic concentrations of the compound of formula (I) according to the invention were measured by LC-MS/MS. The pharmacokinetics parameters were calculated for mean concentrations by non-compartmental model with Phoenix software version 8.1.

### Results

The mean plasma concentration of compound of formula (I) as a function of time following the administration of the composition according to the invention prepared in Example 1 is shown in **Figure 1****.** As it can be seen in **Figure 1****,** the plasma concentration of the compound of formula (I) decreased over 8 hours.

The pharmacokinetics parameters were as follows:

| | **Oral administration (composition A, dose (PO) = 150 mg/kg)** | **Intravenous administration (composition IV, dose (IV) = 1 mg/kg)** |
|---|---|---|
| **Pharmacokinetic parameter** | **Value ± Standard deviation** | **Value ± Standard deviation** |
| Cmax (ng/mL) | 36509.65 ± 3601.20 | |
| Tmax (h) | 1.33 ± 0.58 | |
| T1/2 (h) | 3.33 ± 0.94 | 0.78 ± 0.25 |
| Vd F (L/kg) | 2.91 ± 0.50 | 0.72 ± 0.40 |
| Cl F (mL/min/kg) | 10.32 ± 1.25 | 10.25 ± 2.75 |
| AUC0-last (ng.h/mL) | 193697.22 ± 6026.97 | 1694.97 ± 404.47 |
| AUCO-inf (ng.h/mL) | 244456.55 ± 28592.08 | 1697.48 ± 404.50 |
| Bioavailability F (%) | 96.01 ± 11.23 | |

The bioavailability of the compound (I) was of 96%. Therefore, the composition A according to the invention allows a bioavailability of the compound (I) of more than 90% after one administration by oral route.

### Example 3: Efficacy of the composition A according to the invention prepared in Example 1 in the treatment of colorectal cancer

### Materials and Methods

Tumor cells implantation: 5×10⁵ CT26 cells were implanted subcutaneously onto the flank of 20 female BALB/c mice. When tumors reached approximately 50-100 mm³, mice with similar-sized tumors were randomly assigned to treatment groups (either "test group" or "control group"). The treatments (composition according to the invention prepared in Example 1 for test group or placebo for the control group) started 7 days after tumor cells implantation. All preparations of dosing solutions were carried out in a sterile biosafety cabinet.

The composition A according to the invention prepared in Example 1 was administered every day during 13 days (starting from day 1, by oral route, at a dosage of 150 mg/kg for each mouse (corresponding to a volume dosage of 10 mL/kg by oral route), to 10 female BALB/C mice having a CT26 tumor (corresponding to the "test group").

A vehicle (comprising 50% v/v of Kolliphor^{®} HS15, 33.33% v/v of PEG400 and 16.67% v/v of ethanol, the percentages being expressed relative to the total volume of the vehicle) was administered every day during 13 days (starting from day 1), by oral route, to 10 other female BALB/C mice having a CT26 tumor, as a placebo (corresponding to the "control group").

The follow-up for every mouse comprised: bodyweight measurement twice per week, daily clinical observation (general signs/symptoms), tumor volume measurements 3 times per week, tumor analysis, blood analysis.

The tumors were measured using digital calipers. The length, width and depth of the tumor were measured and used to calculate the tumor volume.

In addition, the tumors were weighed at day 13 after removing them from the mice's bodies.

### Results

The tumor volume as a function of time following the administration of the composition prepared in Example 1 or the placebo is shown in **Figure 2****.** As it can be seen in **Figure 2****,** the oral administration of the composition according to the invention prepared in Example 1 resulted in a threefold reduction in tumor volume at day 13, compared to the control group.

In addition, the mean tumor weight, at day 13, was of 970 mg for the control group and 341 mg for the test group. Thus, the administration of a composition according to the invention, by oral route, allowed a reduction of about 3 times of CT26 tumors in BALB/c mice, as compared to the control group.

Moreover, body weight measurements showed no statistically significant difference between the control and test groups, demonstrating that the oral administration of the composition according to the invention prepared in Example 1 is well tolerated.

### Example 4: Comparison of the bioavailability of the compound of formula (I) after administration of a composition according to the invention and a composition according to the prior art

### Materials and Methods

### Administration by oral route

Two compositions were prepared:
Composition F according to the invention: an organic vehicle was prepared, comprising: 50% v/v of Kolliphor^{®} HS15, 33.33% v/v of PEG400 and 16.67% v/v of ethanol. Thus, in 1.940 mL of said organic vehicle, there was 0.9700 mL of Kolliphor^{®} HS15, 0.6466 mL of PEG400 and 0.3234 mL of ethanol. The compound of formula (I) according to the invention was weighed on a microbalance into a Wheaton vial. The compound was dissolved in 1.9400 mL of the organic vehicle described above and the dosing solution was stirred well at 800 rpm at 25°C using a magnetic bar for approximately 30 minutes. Following this, the final vehicle component, 4.5266 mL of ultrapure water, was added slowly to the Wheaton vial and mixed well by inversion. The final composition, named composition F, contained 15% v/v of Kolliphor^{®} HS15, 10% v/v of PEG400, 5% v/v of ethanol and 70% v/v of ultrapure water, and 0.3 mg of the compound of formula (I) per milliliter of the composition F.

Composition E according to the prior art: the compound of formula (I) according to the invention was added in a mixture containing 1.2% v/v of methylcellulose, 0.1% v/v of Tween^{®} 80 (polyethylene glycol sorbitan monooleate, CAS number: 9005-65-6), qsp 100% v/v of deionized water. The obtained suspension, named composition E, comprised 5 mg of the compound of formula (I) per milliliter of the composition E.

### Administration:

The composition F according to the invention was administered as a single dose by oral route at a dosage of 150 mg of compound of formula (I) per kilogram for each mouse to 3 healthy male swiss albino mice.

The composition E according to the prior art was administered as a single dose by oral route at a dosage of 150 mg of compound of formula (I) per kilogram for each mouse to 3 healthy male swiss albino mice.

Blood sampling: for each mouse, blood samples of approximatively 40 µL to 50 µL per sample were collected by saphenous vein puncture at different endpoints (T0+0.25 hour, T0+0.5 hour, T0+1 hour, T0+2 hours, T0+4 hours and T0+8 hours following the oral administration at T0); blood was be collected into collection tubes with anticoagulant (lithium heparin); tubes were centrifuged (2000 g, 10 minutes, 4°C) to obtain plasma. Samples were stored in propylene tubes at <-65°C until LC-MS/MS analysis (n=126). Pharmacokinetic parameters were calculated from non-compartmental analysis (NCA) of PK profiles using Phoenix WinNonlin 7.0 software.

### Results

The pharmacokinetics parameters after oral administration were as follows:

| **Pharmacinetic parameter** | **Value** | **SD** | **Value** | **SD** |
|---|---|---|---|---|
| **Formulation** | **E** (according to the prior art) | | **F** (according to the invention) | |
| Oral dose (mg/kg) | 150 | | | |
| Cmax (ng/mL) | 2185 | 1438 | 13867 | 2558 |
| Cmax (µmol/L) | 4.69 | 3.09 | 29.7502775 | 5.49 |
| Tmax (h) | 0.5 to 2 | - | 0.5 to 1 | - |
| T1/2 (h) | 3.40 | 2.60 | 2.20 | 0.70 |
| Vd_F_(L/Kg) | 100.20 | 26.10 | 9.8 | 2.8 |
| Cl_F_(mL/min/Kg) | 428.70 | 194.90 | 53.8 | 17.0 |
| AUC_{0-inf} (ng.h/mL) | 7165 | 3212 | 53010 | 14910 |
| Bioavailability (%) | 2.81 | 1.26 | 62.46 | 17.57 |

As it can be seen, after a single oral administration of the composition F according to the invention, the maximum concentration Cmax of the compound of formula (I) according to the invention is obtained between 30 minutes and 1 hour after the administration; it is of 29.75 µM for an administered dose of 150 mg/kg. After a single oral administration of the composition E according to the prior art, the maximum concentration Cmax of the compound of formula (I) according to the invention is obtained between 30 minutes and 2 hours after the administration; it is of 4.69 µM for a dose of 150 mg/kg. Thus, the maximum concentration Cmax is greater after the oral administration of the composition F according to the invention than after the oral administration of the composition E according to the prior art.

In view of the results, it can be concluded that the composition F according to the invention allows a faster absorption (see Tmax) and better exposure (see AUC_{0-inf}) to the compound of formula (I) than the composition E according to the prior art.

In addition, the oral bioavailability is about 62.46 % after a single oral administration of the composition F according to the invention and about 2.81 % after a single oral administration of the composition E according to the prior art.

Therefore, the composition F according to the invention allows a better oral bioavailability of the compound of formula (I) than the composition E according to the prior art.

## Claims

1. A pharmaceutical composition comprising:
- a compound of formula (I) or a pharmaceutically acceptable salt, solvate or mixtures thereof, wherein formula (I) is as follows: 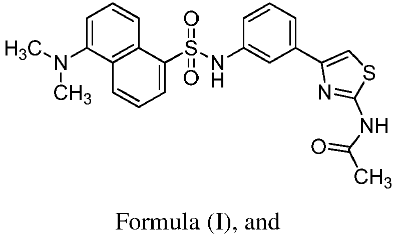
- from 5% v/v to 30% v/v, preferably from 10% v/v to 25% v/v, more preferably from 15% v/v to 25% v/v, even more preferably about 25% v/v, of at least one poly(ethylene glycol) selected from the group consisting of substituted poly(ethylene glycol)s, non-substituted poly(ethylene glycol)s and mixtures thereof, the percentages being expressed relative to the total volume of the pharmaceutical composition,
- from 2% v/v to 10% v/v, preferably from 3% v/v to 7% v/v, more preferably from 4% v/v to 6% v/v, even more preferably about 5% v/v, of at least one solvent selected from the group consisting of ethanol, dimethyl sulfoxide, dimethylacetamide and mixtures thereof, the percentages being expressed relative to the total volume of the pharmaceutical composition, and
- water.

2. The pharmaceutical composition according to claim **1,** wherein the at least one poly(ethylene glycol) is a mixture of a substituted poly(ethylene glycol) and a non-substituted poly(ethylene glycol); preferably, the at least one poly(ethylene glycol) is a mixture of poly(ethylene glycol)(15)-hydroxystearate and poly(ethylene glycol) 400.

3. The pharmaceutical composition according to claim **2,** wherein the non-substituted poly(ethylene glycol) represents from 5% v/v to 15% v/v, preferably from 7% v/v to 13% v/v, more preferably from 8% v/v to 12% v/v, even more preferably about 10% v/v, relative to the total volume of the pharmaceutical composition.

4. The pharmaceutical composition according to claim **2** or **3,** wherein the substituted poly(ethylene glycol) represents from 10% v/v to 20% v/v, preferably from 12% v/v to 18% v/v, more preferably from 14% v/v to 16% v/v, even more preferably about 15% v/v, relative to the total volume of the pharmaceutical composition.

5. The pharmaceutical composition according to any one of claims **1** to **4,** wherein said pharmaceutical composition comprises from 60% v/v to 93% v/v, preferably from 68% v/v to 87% v/v, more preferably from 69% v/v to 81% v/v, even more preferably about 70% v/v, of water.

6. The pharmaceutical composition according to any one of claims **1** to **5,** wherein said pharmaceutical composition comprises from 1 mg/mL to 320 mg/mL of the compound of formula (I) or pharmaceutically acceptable salt, solvate or mixtures thereof, preferably from 3 mg/mL to 20 mg/mL of the compound of formula (I) or pharmaceutically acceptable salt, solvate or mixtures thereof, more preferably about 15 mg/mL of the compound of formula (I) or pharmaceutically acceptable salt, solvate or mixtures thereof.

7. The pharmaceutical composition according to any one of claims **1** to **6,** wherein the at least one solvent is ethanol.

8. The pharmaceutical composition according to any one of claims **1** to **7,** wherein said pharmaceutical composition further comprises at least one further excipient selected from the group consisting of: solvents, diluents carriers, fillers, bulking agents, binders, disintegrants, polymer, lubricant, glidant, surfactants, isotonic agents, thickening or emulsifying agents, stabilizers, absorption accelerators, flavoring agents, taste maskers, preservatives, antioxidants, buffering agents, gelling agent, solubilizing agent and combinations thereof.

9. The pharmaceutical composition according to any one of claims **1** to **8,** for use as a medicament.

10. The pharmaceutical composition according to any one of claims **1** to **8,** for use in the treatment or prevention of a cancer in a subject in need thereof.

11. The pharmaceutical composition for use according to claim **10,** wherein said cancer is a solid cancer, preferably a solid cancer selected from the group consisting of: esophageal cancer, gastrointestinal cancer, gastric cancer, intestinal cancer, colorectal cancer, colon cancer, rectal cancer, pancreatic cancer, biliary tract cancer, liver cancer, kidney cancer, lung cancer, pleural cancer, urinary tract cancer, prostate cancer, endometrium cancer, ovarian cancer, breast cancer, autonomic ganglia cancer, salivary cancer, thyroid cancer, central neural system cancer, bone cancer, soft tissue cancer, lymphoma, skin cancer, carcinoma and melanoma, more preferably a solid cancer selected from the group consisting of: esophageal cancer, gastric cancer, intestinal cancer, colorectal cancer, colon cancer, rectal cancer, and pancreatic cancer, even more preferably a solid cancer selected from the group consisting of: colorectal cancer, colon cancer and rectal cancer.

12. The pharmaceutical composition for use according to claim **10,** wherein said cancer is a liquid cancer, preferably a liquid cancer selected from the group consisting of: lymphoma, leukemia and hematopoietic cancer.

13. The pharmaceutical composition for use according to any one of claims **9** to **12,** wherein said pharmaceutical composition is administered by a route of administration selected from the group consisting of: oral route, intraperitoneal route, intraspinal route, intraarterial route, intravenous route, intramuscular route and subcutaneous route, preferably said pharmaceutical composition is administered by oral route.

14. The pharmaceutical composition for use according to any one of claims **9** to **13,** wherein said pharmaceutical composition is administered once a day, twice a day, three times a day or four times a day.

15. The pharmaceutical composition for use according to any one of claims **9** to **14,** wherein the dosage of the compound of formula (I) or a pharmaceutically acceptable salt, solvate or mixtures thereof, which is administered in one administration to the subject, ranges from 5 mg to 250 mg per kilogram of the subject weight, preferably from 5 mg to 150 mg per kilogram of the subject weight, more preferably from 5 mg to 50 mg per kilogram of the subject weight, even more preferably from 15 mg to 35 mg per kilogram of the subject weight.

## Patentansprüche

1. Pharmazeutische Zusammensetzung, umfassend:
- eine Verbindung der Formel (I) oder ein pharmazeutisch annehmbares Salz, Solvat oder Mischungen davon, wobei die Formel (I) wie folgt ist: 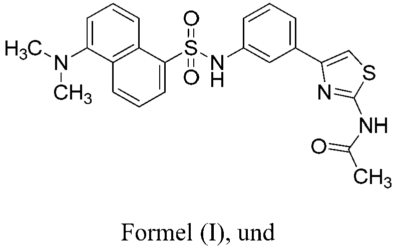
- von 5 % v/v bis 30 % v/v, vorzugsweise von 10 % v/v bis 25 % v/v, bevorzugter von 15 % v/v bis 25 % v/v, noch bevorzugter etwa 25 % v/v, mindestens eines Poly(ethylenglycols), ausgewählt aus der Gruppe bestehend aus substituierten Poly(ethylenglycolen), nicht substituierten Poly(ethylenglycolen) und Mischungen davon, wobei die Prozentangaben auf das Gesamtvolumen der pharmazeutischen Zusammensetzung bezogen sind,
- von 2 % v/v bis 10 % v/v, vorzugsweise von 3 % v/v bis 7 % v/v, bevorzugter von 4 % v/v bis 6 % v/v, noch bevorzugter etwa 5 % v/v, mindestens eines Lösungsmittels, ausgewählt aus der Gruppe bestehend aus Ethanol, Dimethylsulfoxid, Dimethylacetamid und Mischungen davon, wobei die Prozentangaben auf das Gesamtvolumen der pharmazeutischen Zusammensetzung bezogen sind, und
- Wasser.

2. Pharmazeutische Zusammensetzung nach Anspruch 1, wobei das mindestens eine Poly(ethylenglycol) eine Mischung aus einem substituierten Poly(ethylenglycol) und einem nicht substituierten Poly(ethylenglycol) ist; wobei vorzugsweise das mindestens eine Poly(ethylenglycol) eine Mischung aus Poly(ethylenglycol)(15)-hydroxystearat und Poly(ethylenglycol) 400 ist.

3. Pharmazeutische Zusammensetzung nach Anspruch 2, wobei das nicht substituierte Poly(ethylenglycol) von 5 % v/v bis 15 % v/v, vorzugsweise von 7 % v/v bis 13 % v/v, bevorzugter von 8 % v/v bis 12 % v/v, noch bevorzugter etwa 10 % v/v, bezogen auf das Gesamtvolumen der pharmazeutischen Zusammensetzung, ausmacht.

4. Pharmazeutische Zusammensetzung nach Anspruch 2 oder 3, wobei das substituierte Poly(ethylenglycol) von 10 % v/v bis 20 % v/v, vorzugsweise von 12 % v/v bis 18 % v/v, bevorzugter von 14 % v/v bis 16 % v/v, noch bevorzugter etwa 15 % v/v, bezogen auf das Gesamtvolumen der pharmazeutischen Zusammensetzung, ausmacht.

5. Pharmazeutische Zusammensetzung nach einem der Ansprüche 1 bis 4, wobei die pharmazeutische Zusammensetzung von 60 % v/v bis 93 % v/v, vorzugsweise von 68 % v/v bis 87 % v/v, bevorzugter von 69 % v/v bis 81 % v/v, noch bevorzugter etwa 70 % v/v, Wasser umfasst.

6. Pharmazeutische Zusammensetzung nach einem der Ansprüche 1 bis 5, wobei die pharmazeutische Zusammensetzung von 1 mg/mL bis 320 mg/mL der Verbindung der Formel (I) oder eines pharmazeutisch annehmbaren Salzes, Solvats oder von Mischungen davon, vorzugsweise von 3 mg/mL bis 20 mg/mL der Verbindung der Formel (I) oder eines pharmazeutisch annehmbaren Salzes, Solvats oder von Mischungen davon, bevorzugter etwa 15 mg/mL der Verbindung der Formel (I) oder eines pharmazeutisch annehmbaren Salzes, Solvats oder von Mischungen davon umfasst.

7. Pharmazeutische Zusammensetzung nach einem der Ansprüche 1 bis 6, wobei das mindestens eine Lösungsmittel Ethanol ist.

8. Pharmazeutische Zusammensetzung nach einem der Ansprüche 1 bis 7, wobei die pharmazeutische Zusammensetzung ferner mindestens einen weiteren Hilfsstoff umfasst, ausgewählt aus der Gruppe bestehend aus: Lösungsmitteln, Verdünnungsmitteln Trägern, Füllstoffen, Volumenbildnern, Bindemitteln, Sprengmitteln, Polymer, Schmiermittel, Fließregulierungsmittel, Tensiden, Isotonisierungsmitteln, Verdickungs- oder Emulgiermitteln, Stabilisatoren, Absorptionsbeschleunigern, Aromastoffen, Geschmacksmaskierungsmitteln, Konservierungsmitteln, Antioxidantien, Puffermitteln, Geliermittel, Lösungsvermittler und Kombinationen davon.

9. Pharmazeutische Zusammensetzung nach einem der Ansprüche 1 bis 8 zur Verwendung als Arzneimittel.

10. Pharmazeutische Zusammensetzung nach einem der Ansprüche 1 bis 8 zur Verwendung bei der Behandlung oder Vorbeugung einer Krebserkrankung bei einem Subjekt, das dessen bedarf.

11. Pharmazeutische Zusammensetzung zur Verwendung nach Anspruch 10, wobei die Krebserkrankung eine solide Krebserkrankung ist, vorzugsweise eine solide Krebserkrankung, ausgewählt aus der Gruppe bestehend aus: Speiseröhrenkrebs, Magen-Darm-Krebs, Magenkrebs, Darmkrebs, kolorektalem Krebs, Kolonkrebs, Rektumkrebs, Bauchspeicheldrüsenkrebs, Gallenwegskrebs, Leberkrebs, Nierenkrebs, Lungenkrebs, Pleurakrebs, Harnwegskrebs, Prostatakrebs, Endometriumkrebs, Eierstockkrebs, Brustkrebs, Krebs der autonomen Ganglien, Speicheldrüsenkrebs, Schilddrüsenkrebs, Krebs des zentralen Nervensystems, Knochenkrebs, Weichteilkrebs, Lymphom, Hautkrebs, Karzinom und Melanom, bevorzugter eine solide Krebserkrankung, ausgewählt aus der Gruppe bestehend aus: Speiseröhrenkrebs, Magenkrebs, Darmkrebs, kolorektalem Krebs, Kolonkrebs, Rektumkrebs und Bauchspeicheldrüsenkrebs, noch bevorzugter eine solide Krebserkrankung, ausgewählt aus der Gruppe bestehend aus: kolorektalem Krebs, Kolonkrebs und Rektumkrebs.

12. Pharmazeutische Zusammensetzung zur Verwendung nach Anspruch 10, wobei die Krebserkrankung eine flüssige Krebserkrankung ist, vorzugsweise eine flüssige Krebserkrankung, ausgewählt aus der Gruppe bestehend aus: Lymphom, Leukämie und hämatopoetischem Krebs.

13. Pharmazeutische Zusammensetzung zur Verwendung nach einem der Ansprüche 9 bis 12, wobei die pharmazeutische Zusammensetzung über einen Verabreichungsweg verabreicht wird, der ausgewählt ist aus der Gruppe bestehend aus: oralem Weg, intraperitonealem Weg, intraspinalem Weg, intraarteriellem Weg, intravenösem Weg, intramuskulärem Weg und subkutanem Weg, wobei die pharmazeutische Zusammensetzung vorzugsweise auf oralem Weg verabreicht wird.

14. Pharmazeutische Zusammensetzung zur Verwendung nach einem der Ansprüche 9 bis 13, wobei die pharmazeutische Zusammensetzung einmal täglich, zweimal täglich, dreimal täglich oder viermal täglich verabreicht wird.

15. Pharmazeutische Zusammensetzung zur Verwendung nach einem der Ansprüche 9 bis 14, wobei die Dosis der Verbindung der Formel (I) oder eines pharmazeutisch annehmbaren Salzes, Solvats oder von Mischungen davon, die bei einer Verabreichung an das Subjekt verabreicht wird, im Bereich von 5 mg bis 250 mg pro Kilogramm Körpergewicht des Subjekts liegt, vorzugsweise von 5 mg bis 150 mg pro Kilogramm Körpergewicht des Subjekts, bevorzugter von 5 mg bis 50 mg pro Kilogramm Körpergewicht des Subjekts, noch bevorzugter von 15 mg bis 35 mg pro Kilogramm Körpergewicht des Subjekts.

## Revendications

1. Composition pharmaceutique comprenant :
- un composé de formule (I) ou un sel ou solvate pharmaceutiquement acceptable ou des mélanges de ceux-ci, la formule (I) étant la suivante : 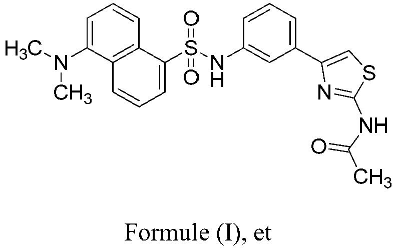
- de 5 % v/v à 30 % v/v, de préférence de 10 % v/v à 25 % v/v, plus préférentiellement de 15 % v/v à 25 % v/v, encore plus préférentiellement environ 25 % v/v, d'au moins un poly(éthylène glycol) choisi dans le groupe constitué des poly(éthylène glycol)s substitués, des poly(éthylène glycol)s non substitués et de leurs mélanges, les pourcentages étant exprimés par rapport au volume total de la composition pharmaceutique,
- de 2 % v/v à 10 % v/v, de préférence de 3 % v/v à 7 % v/v, plus préférentiellement de 4 % v/v à 6 % v/v, encore plus préférentiellement environ 5 % v/v, d'au moins un solvant choisi dans le groupe constitué de l'éthanol, du diméthylsulfoxyde, du diméthylacétamide et de leurs mélanges, les pourcentages étant exprimés par rapport au volume total de la composition pharmaceutique, et
- de l'eau.

2. Composition pharmaceutique selon la revendication 1, dans laquelle l'au moins un poly(éthylène glycol) est un mélange d'un poly(éthylène glycol) substitué et d'un poly(éthylène glycol) non substitué ; de préférence, l'au moins un poly(éthylène glycol) est un mélange de poly(éthylène glycol)(15)-hydroxystéarate et de poly(éthylène glycol) 400.

3. Composition pharmaceutique selon la revendication 2, dans laquelle le poly(éthylène glycol) non substitué représente de 5 % v/v à 15 % v/v, de préférence de 7 % v/v à 13 % v/v, plus préférentiellement de 8 % v/v à 12 % v/v, encore plus préférentiellement environ 10 % v/v, par rapport au volume total de la composition pharmaceutique.

4. Composition pharmaceutique selon la revendication 2 ou 3, dans laquelle le poly(éthylène glycol) substitué représente de 10 % v/v à 20 % v/v, de préférence de 12 % v/v à 18 % v/v, plus préférentiellement de 14 % v/v à 16 % v/v, encore plus préférentiellement environ 15 % v/v, par rapport au volume total de la composition pharmaceutique.

5. Composition pharmaceutique selon l'une quelconque des revendications 1 à 4, ladite composition pharmaceutique comprenant de 60 % v/v à 93 % v/v, de préférence de 68 % v/v à 87 % v/v, plus préférentiellement de 69 % v/v à 81 % v/v, encore plus préférentiellement environ 70 % v/v, d'eau.

6. Composition pharmaceutique selon l'une quelconque des revendications 1 à 5, ladite composition pharmaceutique comprenant de 1 mg/mL à 320 mg/mL du composé de formule (I) ou d'un sel ou solvate pharmaceutiquement acceptable ou de mélanges de ceux-ci, de préférence de 3 mg/mL à 20 mg/mL du composé de formule (I) ou d'un sel ou solvate pharmaceutiquement acceptable ou de mélanges de ceux-ci, plus préférentiellement environ 15 mg/mL du composé de formule (I) ou d'un sel ou solvate pharmaceutiquement acceptable ou de mélanges de ceux-ci.

7. Composition pharmaceutique selon l'une quelconque des revendications 1 à 6, dans laquelle l'au moins un solvant est l'éthanol.

8. Composition pharmaceutique selon l'une quelconque des revendications 1 à 7, ladite composition pharmaceutique comprenant en outre au moins un autre excipient choisi dans le groupe constitué : des solvants, diluants supports, charges, agents de masse, liants, délitants, polymère, lubrifiant, agent d'écoulement, tensioactifs, agents d'isotonisité, agents épaississants ou émulsifiants, stabilisants, accélérateurs d'absorption, agents aromatisants, agents masquant le goût, conservateurs, antioxydants, agents tampons, agent gélifiant, agent solubilisant et de leurs combinaisons.

9. Composition pharmaceutique selon l'une quelconque des revendications 1 à 8, pour son utilisation en tant que médicament.

10. Composition pharmaceutique selon l'une quelconque des revendications 1 à 8, pour son utilisation dans le traitement ou la prévention d'un cancer chez un sujet qui en a besoin.

11. Composition pharmaceutique pour son utilisation selon la revendication 10, dans laquelle ledit cancer est un cancer solide, de préférence un cancer solide choisi dans le groupe constitué : du cancer de l'œsophage, du cancer gastro-intestinal, du cancer gastrique, du cancer intestinal, du cancer colorectal, du cancer du côlon, du cancer du rectum, du cancer du pancréas, du cancer des voies biliaires, du cancer du foie, du cancer du rein, du cancer du poumon, du cancer de la plèvre, du cancer des voies urinaires, du cancer de la prostate, du cancer de l'endomètre, du cancer de l'ovaire, du cancer du sein, du cancer des ganglions autonomes, du cancer des glandes salivaires, du cancer de la thyroïde, du cancer du système nerveux central, du cancer des os, du cancer des tissus mous, du lymphome, du cancer de la peau, du carcinome et du mélanome, plus préférentiellement un cancer solide choisi dans le groupe constitué : du cancer de l'œsophage, du cancer gastrique, du cancer intestinal, du cancer colorectal, du cancer du côlon, du cancer du rectum et du cancer du pancréas, encore plus préférentiellement un cancer solide choisi dans le groupe constitué : du cancer colorectal, du cancer du côlon et du cancer du rectum.

12. Composition pharmaceutique pour son utilisation selon la revendication 10, dans laquelle ledit cancer est un cancer liquide, de préférence un cancer liquide choisi dans le groupe constitué : du lymphome, de la leucémie et du cancer hématopoïétique.

13. Composition pharmaceutique pour son utilisation selon l'une quelconque des revendications 9 à 12, dans laquelle ladite composition pharmaceutique est administrée par une voie d'administration choisie dans le groupe constitué : de la voie orale, de la voie intrapéritonéale, de la voie intrarachidienne, de la voie intra-artérielle, de la voie intraveineuse, de la voie intramusculaire et de la voie sous-cutanée, de préférence ladite composition pharmaceutique est administrée par voie orale.

14. Composition pharmaceutique pour son utilisation selon l'une quelconque des revendications 9 à 13, dans laquelle ladite composition pharmaceutique est administrée une fois par jour, deux fois par jour, trois fois par jour ou quatre fois par jour.

15. Composition pharmaceutique pour son utilisation selon l'une quelconque des revendications 9 à 14, dans laquelle la dose du composé de formule (I) ou d'un sel ou solvate pharmaceutiquement acceptable ou de mélanges de ceux-ci, qui est administrée en une administration au sujet, va de 5 mg à 250 mg par kilogramme de poids du sujet, de préférence de 5 mg à 150 mg par kilogramme de poids du sujet, plus préférentiellement de 5 mg à 50 mg par kilogramme de poids du sujet, encore plus préférentiellement de 15 mg à 35 mg par kilogramme de poids du sujet.
